Europäisches Patentamt

European Patent Office

Office européen des brevets

(19)

(11) Veröffentlichungsnummer: **0 404 161 B1**

(12) **EUROPÄISCHE PATENTSCHRIFT**

(45) Veröffentlichungstag der Patentschrift: **29.03.95**

(51) Int. Cl.⁶: **C12Q 1/68**, C07H 21/04

(21) Anmeldenummer: **90111779.6**

(22) Anmeldetag: **21.06.90**

Die Akte enthält technische Angaben, die nach dem Eingang der Anmeldung eingereicht wurden und die nicht in dieser Patentschrift enthalten sind.

(54) **Neisseria-spezifische DNA-Sonden.**

(30) Priorität: **22.06.89 DE 3920492**
**02.08.89 DE 3925613**

(43) Veröffentlichungstag der Anmeldung:
**27.12.90 Patentblatt 90/52**

(45) Bekanntmachung des Hinweises auf die Patenterteilung:
**29.03.95 Patentblatt 95/13**

(84) Benannte Vertragsstaaten:
**AT BE CH DE DK ES FR GB GR IT LI LU NL SE**

(56) Entgegenhaltungen:
**EP-A- 0 238 332**
**WO-A-89/03891**

**CLINICAL MICROBIOLOGY REVIEWS, April 1989; pp. 564-565&NUM;**

(73) Patentinhaber: **BOEHRINGER MANNHEIM GMBH**

**D-68298 Mannheim (DE)**

(72) Erfinder: **Stern, Anne, Dr.**
**Karwendelstrasse 10**
**D-8122 Penzberg (DE)**
Erfinder: **Wolff, Karin**
**Friedlandstrasse 23**
**D-8034 Germering (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**Beschreibung**

Die Erfindung betrifft Neisseria-spezifische DNA-Sonden und ein Verfahren zum Nachweis wenigstens einer der pathogenen Neisseria-Spezies N. gonorrhoeae und N. meningitidis.

Die Gattung Neisseria stellt eine Gruppe eng verwandter, Gram-negativer Diplokokken dar, zu der sowohl pathogene wie auch apathogene Spezies gehören.

Die pathogenen Spezies sind N. gonorrhoeae und N. meningitidis. Daneben gibt es einige nicht-pathogene Arten, die Bestandteil der physiologischen Flora des oberen Respirationstraktes sind.

N. gonorrhoeae ist der Erreger der Gonorrhoe, der auch heute noch häufigsten meldepflichtigen Infektionskrankheit der Welt (World Health Statistics Annual. (1979), Geneva, WHO). Allein in den USA werden pro Jahr mehrere Millionen Fälle registriert; bei fehlender Behandlung dieser Krankheit kann es zu ernsthaften Folgeerkrankungen kommen, wie z.B. Prostatitis, Peritonitis und Arthritis. Die Infektionen verlaufen häufig zunächst asymptomatisch, wodurch viele Träger unwissentlich zur Verbreitung der Krankheit beitragen.

Die bisher zur Diagnose der Krankheit herangezogene Methode hängt vom Geschlecht des Patienten und den Symptomen ab: Anlegen einer Kultur, welche 24 bis 48 Stunden inkubieren muß und Austesten der kultivierten Organismen über die Gram-Färbungsmethode, den Kohlehydrat-Abbau, Coagglutination, Fluoreszenz-Antikörper-Screening etc. Bei einer disseminierten Gonorrhoe sollten Blutkulturen angelegt werden bzw. müssen Proben von den befallenen Regionen gewonnen werden, ein insgesamt sehr aufwendiges Vorgehen.

N. meningitidis ist einer der Erreger der bakteriellen Hirnhautentzündung. Etwa 20 % aller übertragbaren Meningitiden werden durch Meningokokken hervorgerufen. Voraussetzung für den Ausbruch der Krankheit ist die Besiedelung des Rachenraumes mit virulenten Meningokocken (vorwiegend der Serogruppen A, B oder C) sowie das Fehlen ausreichender Abwehrkräfte des Immunsystems.

Eine sichere Diagnose der Meningitis ist bisher nur bakteriologisch möglich. Ein entscheidender Schritt ist dabei wiederum das Anlegen einer Kultur. Darüberhinaus führt man zur Abgrenzung gegenüber anderen Gram-negativen, Oxidase-positiven Kokken eine Differentialdiagnose durch: Austesten der Zuckerverwertung, Agglutinationsreaktionen mit Serogruppen-spezifischen Antikörpern etc.

Der diagnostische Nachweis der beiden pathogenen Neisseria-Spezies setzt also in den überwiegenden Fällen das Anlegen einer Kultur voraus, die mindestens 24 Stunden Inkubationszeit erfordert. Hinzu kommt, daß die Anzucht - besonders von N. gonorrhoeae - sehr schwierig ist. Zur Kultivierung beider Spezies werden Selektivmedien herangezogen, auf denen jedoch auch apathogene Neisserien wie z.B. N. lactamica wachsen können. Aus diesem Grund besteht die Gefahr einer Verwechslung, die nur durch exakte und aufwendige biochemische Differenzierung ausgeschlossen werden kann. Es wurde daher bereits versucht, Diagnosetests zu entwickeln, die schnell und spezifisch den Nachweis der pathogenen Neisseria-Spezies erlauben. Eine Möglichkeit hierzu schien die sich vom Gesichtspunkt der Schnelligkeit her anbietende Technik der Nukleinsäure-Hybridisierung zu sein. Es wurde daher für N. gonorrhoeae bereits versucht, Nachweisverfahren mit Hilfe dieser Technik zu etablieren, wobei man sich vor allem auf die folgenden Nukleinsäure-Sonden konzentrierte: Sequenzen, die komplementär zu rRNAs sind (EP-A 0 272 009), Sequenzen auf kryptischen Plasmiden, die vorrangig bei N. gonorrhoeae vorkommen (Totten et al., J. Inf. Dis. 148 (1983), 462 und Perin et al., J. Inf. Dis. 152 (1986), 59) und Sequenzen unbekannter Funktion, die mittels aufwendiger Screening-Methoden gefunden werden können (EP-A 0 237 737).

Für all diese Ansätze stellt sich jedoch das Problem, daß die Gattung Neisseria eine Gruppe eng verwandter Organismen darstellt, die genetisch schwer voneinander zu unterscheiden sind. Der Grad der genetischen Verwandtschaft ist besonders zwischen N. gonorrhoeae und N. meningitidis außergewöhnlich hoch. So weisen DNA/DNA-Hybridisierungen der chromosomalen DNAs auf einen Homologiegrad von 93 % hin (Hoke und Vedros, Ing. J. Sys. Bact. 32 (1982), 57). Die auffallend starke Homologie bleibt jedoch nicht auf die pathogene Neisseria-Spezies beschränkt, sondern vielmehr konnte z.B. durch Transformations-Experimente und DNA-Hybridisierungen gezeigt werden, daß N. meningitidis und N. lactamica ebenfalls eng miteinander verwandt sind.

Aufgabe der vorliegenden Erfindung war es daher, spezifische Nachweis-Sonden bereitzustellen, welche es erlauben, über die Technik der DNA-Hybridisierung pathogene von apathogenen Neisseria-Spezies zu unterscheiden und somit sowohl einen qualitativen als auch einen quantitativen Nachweis der pathogenen Spezies zu ermöglichen.

Erfindungsgemäß wird diese Aufgabe gelöst durch eine für pathogene Neisseria-Spezies spezifische DNA-Sonde, welche ausgewählt ist aus der Gruppe der Oligonukleotide Opa 1, Opa 2, Opa 3, PIII, Pil 1, IgA 1 und IgA 2. Die DNA-Sequenzen der erfindungsgemäßen Nukleotide sind in Tabelle I gezeigt.

Tabelle I

| Bezeichnung | Sequenz 5′->3′ |
|---|---|
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| Iga 1 | cggtattacccggt |
| Opa 3 | gcagcccgtattat |

Diese erfindungsgemäßen DNA-Sonden entsprechen bestimmten Teilen von Genen für Neisseria-Proteine, welche nahezu ausschließlich in pathogenen Spezies vorkommen. Dies sind die Proteine IgA 1-Protease (Pohlner et al., Nature 235 (1987), Seite 458), Pilin (Haas et al., Cell 44 (1986), Seite 107, Perry et al., Gene 60 (1987), Seite 85), Protein II (Stern et al., Cell 47 (1986):61, Stern et al., Mol. Microbiol. 1 (1987) 5), Protein III (Gotschlich, J. Exp. Med. 165 (1987), Seite 471).

Manche der Gene für die genannten Proteine wurden bereits in Hybridisierungsstudien eingesetzt, wobei ein Plasmid verwendet wurde, das das gesamte Gen des entsprechenden Proteins und zum Teil auch flankierende Sequenzen des Bakteriengenoms enthielt (Aho et al., Infect. Immun. 55 (1987), Seite 1009, Horn et al., Diagn. Microbiol. Infect. Dis. 4 (1986), Seite 101). Es war jedoch mit keiner dieser Suchproben ein spezifischer Nachweis der pathogenen Spezies und auch keine Unterscheidung der beiden pathogenen Spezies gelungen.

Überraschenderweise gelingt es jedoch mit den erfindungsgemäßen Sonden, welche bestimmten Teilen der genannten Gene entsprechen, außerordentlich spezifisch pathogene von nicht-pathogenen Neisseria-Spezies ebenso wie von non-Neisseria-Spezies zu unterscheiden, und zusätzlich gelingt es mit einigen der Sonden, spezifisch zwischen den beiden Spezies N. gonorrhoeae und N. meningitidis zu unterscheiden.

Die erfindungsgemäßen Sonden sind mindestens 14 Nukleotide lang, und können an ihrem 5′- und/oder 3′-Ende weitere Nukleotide, vorzugsweise bis zu 5 Nukleotide, aufweisen. Eine Gesamtlänge von 40 Nukleotiden sollte nicht überschritten werden.

Die zusätzlichen Nukleotide können hierbei beliebige Nukleotide sein, bevorzugt sind jedoch diese Nukleotide gleich zu den im entsprechenden Protein sich am 5′- bzw. 3′-Ende befindenden Nukleotiden.

Erfindungsgemäß besonders bevorzugte Oligonukleotide, Opa1*, Opa2*, Opa3*, PIII*, Pil1*, IgA1* und IgA2*, sind in der Tabelle II im Beispiel angeführt.

Die erfindungsgemäßen Neisseria-spezifischen DNA-Sonden können weiterhin in verschiedenen Formen vorliegen. So können sie als einzelsträngige Oligonukleotide, mit einem Komplementär-Oligonukleotid hybridisiert als doppelsträngige DNA-Fragmente, oder assoziiert mit anderen Sequenzen, die keine Homologie zur DNA aus Neisseria-Spezies aufweisen, z.B. Klonierungsvektoren, vorliegen. Hierbei ergibt sich wiederum die Möglichkeit zur Unterscheidung in Einzelstrang-Vektoren, wie z.B. M13 und Doppelstrang-Vektoren, wie z.B. die pBR322-Derivate. Weiter können jedoch vorzugsweise auch die einzelnen Oligonukleotide in Einzel- oder Doppelstrangform miteinander gekoppelt werden, so daß zwei oder mehr der Oligonukleotide in einem Vektor vorliegen. Bei Verwendung von Doppelstrangformen wird vor der tatsächlichen Nachweisreaktion durch Denaturierung eine Auftrennung der Sonde in die Einzelstränge bewirkt.

In einer weiteren bevorzugten Ausführungsform der Erfindung sind die Nukleinsäure-Sonden markiert. Dies ist prinzipiell möglich über alle an sich bekannten Arten der Markierung von Nukleinsäuren wie radioaktive Markierung, nämlich den Einbau von radioaktiven Isotopen, oder aber vorzugsweise über nicht radioaktive Markierung, z.B. über den Einbau modifizierter Nukleotide.

Ein weiterer Gegenstand der Erfindung ist ein Verfahren zum Nachweis wenigstens einer der pathogenen Neisseria-Spezies N. gonorrhoeae und N. meningitidis unter Verwendung einer hybridisierenden Sonde bei Hybridisierungsbedingungen und Nachweis der Hybridbildung, das dadurch gekennzeichnet ist, daß man zum spezifischen Nachweis eine der erfindungsgemäßen DNA-Sonden verwendet.

Die erfindungsgemäßen DNA-Sonden hybridisieren sowohl mit Abschnitten der chromosomalen Neisseria-DNA, welche für die Pathogenitätsfaktoren kodieren, als auch mit RNA, nämlich jenem Anteil der mRNA, der das Transkriptionsprodukt der Pathogenitätsfaktor-Gene darstellt. Es ist daher im erfindungsgemäßen Verfahren möglich, An- oder Abwesenheit der pathogenen Neisseria-Spezies quantitativ und qualitativ zu bestimmen. Weiter ist durch das erfindungsgemäße Verfahren auch eine Unterscheidung zwischen den beiden pathogenen Neisseria-Spezies möglich.

EP 0 404 161 B1

Zum Nachweis beider pathogener Spezies werden erfindungsgemäß die Sonden PIII, Pil 1, IgA 2 und Opa 1 und vorzugsweise die Sonden PIII*, Pil1*, IgA2* und Opa1* verwendet, wovon wiederum die Sonden PIII, Pil 1, PIII*, Pil1*, IgA2 und IgA2* besonders bevorzugt sind. Mit den Sonden Opa 1 und Opa1* können, wie aus Tabelle III zu entnehmen ist, sich Hybridisierungen mit nichtpathogenen Neisseria-Spezies in geringem Umfang ergeben. Jedoch sind auch diese beiden Sonden für eine qualitative Untersuchung gut geeignet, wobei bei positivem Testergebnis gegebenenfalls mit einer der anderen Sonden eine Überprüfung durchgeführt werden könnte.

Zum Nachweis von Neisseria gonorrhoeae alleine bzw. für die Unterscheidung von Neisseria meningitidis wird erfindungsgemäß eine der Sonden IgA 1 und Opa 2 und vorzugsweise IgA1* und Opa2* verwendet, wobei IgA 1 und IgA1* wiederum aufgrund der fehlenden Wechselwirkung mit nicht-pathogenen Neisseria-Spezies besonders bevorzugt sind.

Zum Nachweis von N. meningitidis alleine wird erfindungsgemäß die Sonde Opa 3 und vorzugsweise Opa3* verwendet.

Der Nachweis über die Hybridisierung mit mindestens einer der erfindungsgemäßen Sonden kann nach an sich bekannten Methoden zum Nachweis von Nukleinsäuren über Hybridisierung durchgeführt werden. Die DNA-DNA-Hybridisierung wurde erstmals von Southern in J. Mol. Biol. 98 (1975) 503 beschrieben und in der Folge weiterentwickelt. Alle geeigneten Nukleinsäure-Hybridisierungs-Ansätze können Verwendung finden wie z.B. Festphasen-Hybridisierung, Hybridisierung in Lösung, Sandwich-Hybridisierung, Zwei-Komponenten-Hybridisierung. Der Nachweis erfolgt dann über entweder eine radioaktive oder nicht-radioaktive Markierung der Sonde.

Erfindungsgemäß gelingt es durch Verwendung bestimmter Neisseria-spezifischer DNA-Sonden, einen schnellen und sicheren Nachweis des Vorhandenseins pathogener Neisseria-Spezies in Körperflüssigkeiten und Abstrichen und damit einer Infektion des Patienten zu führen. Hierzu ist keine Bakterien-Kultur nötig und auch eine anderweitige Untersuchung zur Abgrenzung gegenüber anderen Bakterien wird vermieden. Es kann vielmehr durch das erfindungsgemäße Verfahren sogar zwischen den nah verwandten pathogenen und nicht- pathogenen Neisseria-Spezies unterschieden werden.

Das folgende Beispiel soll die Erfindung weiter erläutern.

**Beispiel**

Mit Hilfe einer Slot-Blot-Apparatur wurde chromosomale DNA der folgenden Neisseria-Spezies jeweils auf ein Slot der Apparatur auf einen Nitrocellulosefilter aufgebracht:
- pathogene Neisseria-Spezies:
  N. gonorrhoeae: 7 unterschiedliche Isolate,
  N. meningitidis: 7 unterschiedliche Isolate,
- apathogene Neisseria-Spezies:
  N. lactamica, N. mucosa, N. subflava, N. perflava,
  N. sicca, N. elongata, N. cinerea, N. flava, N.
  denidrificans.
- non-Neisseria-Spezies:
  Haemophilus influenzae, Haemophilus parainfluenzae, Streptococcus salivarius, Streptococcus mutans, Streptococcus agalactiae, Staphylococus aureus, Staphylococus epidermidis, Staphylococus saprophyticus, Escherichia coli.

Die Präparation der chromosomalen DNAs erfolgte nach dem von Stern et al., Cell 37 (1984), Seite 447 beschriebenen Protokoll.

Die Filter wurden hierzu in die Apparatur eingespannt, ein Vakuum angelegt und die einzelnen Auftragspunkte mit 200 $\mu$l 2x Puffer angefeuchtet (2x Puffer: 2 mol/l NaCl, 50 mmol/l Tris-HCl, pH 7,5, 1 mmol/l EDTA). Die DNA wurde mit 50 mmol/l Tris-HCl, pH 7,5, und 5 mmol/l EDTA versetzt und zur Denaturierung 3 Minuten lang gekocht.

Anschließend wurde der Ansatz sofort auf Eis überführt, 50 $\mu$l 2x Puffer zugegeben und diese Lösung in die Slots pipettiert. Die Slots wurden mit 100 $\mu$l 1x Puffer (2x Puffer 1:1 mit Wasser verdünnt) nachgespült, der Filter der Apparatur entnommen und im Vakuum getrocknet. Die Hybridisierung erfolgte im wesentlichen nach den bereits beschriebenen Methoden (Southern, J. Mol. Biol. 98 (1975), Seite 503).

Zum Feststellen der Spezies-Spezifität wurden die synthetisierten Nukleinsäure-Sonden gegen die filtergebundene chromosomale DNAs aus den verschiedenen Neisseria-Spezies hybridisiert. Hierzu wurden die Nitrocellulosefilter zwei Stunden in 1xVHP bei 42 °C vorhybridisiert (2xVHP: 0,1 % Rinderserumalbumin, 0,1 % Ficoll 400 000, 0,1 % Polyvinylpropylidon, 1 % Glycerin, 1,8 mol/l NaCl, 50 mmol/l $Na_2HPO_4$ und 10 mg/ml Heringssperm-DNA). Die im VHP enthaltene Heringsspermien-DNA war zuvor durch Erhitzen auf

4

80 °C denaturiert worden.

Die Hybridisierung erfolgte in einem Hybridisierungsofen bei 42 °C. Der Vorhybridisierungspuffer wurde entfernt und durch die $^{32}$P-markierte Oligonukleotid-Probe in Hybridisierungspuffer (HP) ersetzt (HP: 1xVHP und Zugabe von 0 bis 30 % Formamid, je nach GC-Gehalt der Nukleinsäure-Sonde). Der HP wurde vor Verwendung auf 80 °C erhitzt. Nach einer Hybridisierungszeit von mindestens 6 Stunden wurden die Filter in Waschpuffer (WP: 3,6 mol/l NaCl, 100 mmol/l $Na_2HPO_4$, 0,05 % SDS) zunächst zweimal bei Raumtemperatur und dann zweimal bei 42 °C gewaschen. Die Waschtemperatur wurde dann je nach GC-Gehalt der Probe bis auf maximal 68 °C erhöht. Die tatsächlichen maximalen Waschtemperaturen sowie der Formamidgehalt sind auch in Tabelle II dargestellt. Diese Tabelle zeigt außerdem die Sequenzen der verwendeten Nukleinsäure-Sonden.

Tabelle II

| Bezeichnung Sonde | Sequenz 5'->3' | Wasch-Temperatur °C | % Formamid im HP |
|---|---|---|---|
| PIII* | ccatttccctgtctgccaa | 50 | 15 |
| Pil 1* | aaccggctgtccgcagaa | 60 | 30 |
| Iga 2* | cggacggtgcacaaattg | 50 | 15 |
| Opa 1* | ctgcgctgcggaagagaagag | 60 | 30 |
| Opa 2* | gcggcccgtatgtgcagg | 68 | 30 |
| Iga 1* | cggtattacccggttgt | 50 | 15 |
| Opa 3* | gcagcccgtattatgtgc | 48 | 10 |

Die Filter wurden gegen einen Röntgenfilm exponiert.

In Tabelle III sind die Ergebnisse der Hybridisierung der verschiedenen Nukleinsäuresonden mit pathogenen, nicht-pathogenen Neisseria-Spezies und non-Neisseria-Spezies gezeigt.

Tabelle IIIa: Proben, die spezifisch sind für pathogene Neisseria-Species

| Species/Stämme | | Nukleinsäure-Sonden | | | |
|---|---|---|---|---|---|
| | | P III* | Pil 1* | IgA 2* | Opa 1* |
| Neisseria | 74 | + | + | + | + |
| gonorrhoeae | 514 | + | + | + | + |
| | 510 | + | + | + | + |
| | R2 | + | + | + | + |
| | R21 | + | + | + | + |
| | R 16 | + | + | + | + |
| | MS11 | + | + | + | + |
| Neisseria | B | + | + | + | + |
| meningitidis | C | + | + | + | + |
| | D | + | + | + | + |
| | Y | + | + | + | + |
| | W | + | + | + | + |
| | Z | + | + | + | + |
| | 8213 | + | + | + | + |
| Neisseria lactamica | 1855 | − | − | − | + |
| Neisseria lactamica | 2879 | − | − | − | + |
| Neisseria lactamica | 3272 | − | − | − | + |
| Neisseria mucosa | 112 | − | − | − | − |
| Neisseria mucosa | 114 | − | − | − | − |
| Neisseria mucosa | 2888 | − | − | − | − |
| Neisseria subflava | 124 | − | − | − | − |
| Neisseria perflava | 120 | − | − | − | − |
| Neisseria sicca | 118 | − | − | − | − |
| Neisseria sicca | 2844 | − | − | − | − |
| Neisseria elongata | 129 | − | − | − | − |
| Neisseria cinerea | 126 | − | − | − | − |
| Neisseria cinerea | 2199 | − | − | − | − |
| Neisseria flava | 122 | − | − | − | − |
| Neisseria flava | 123 | − | − | − | + |
| Neisseria denitrificans | 2950 | − | − | − | − |
| Haemophilus influenzae | | − | − | − | − |
| Haemophilus parainfluenzae | | − | − | − | − |
| Streptococcus salivarius | | − | − | − | − |
| Streptococcus mutans | | − | − | − | − |
| Streptococcus agalactiae | | − | − | − | − |
| Staphylococus aureus | | − | − | − | − |
| Staphylococus epidermidis | | − | − | − | − |
| Staphylococus saprophyticus | | − | − | − | − |
| Escherichia coli | | − | − | − | − |

Tabelle IIIb: Proben, mit deren Hilfe man zwischen N.gonorrhoeae und N.meningitidis differenzieren kann.

| Species/Stämme | | Nukleinsäure-Sonde (Oligonukleotid) Opa 2* |
|---|---|---|
| Neisseria | 74 | + |
| gonorrhoeae | 514 | + |
| | 510 | + |
| | R2 | + |
| | R21 | + |
| | R 16 | + |
| | MS11 | + |
| Neisseria | B | − |
| meningitidis | C | − |
| | D | − |
| | Y | − |
| | W | − |
| | Z | − |
| | 8213 | − |
| Neisseria lactamica | 1855 | + |
| Neisseria lactamica | 2879 | + |
| Neisseria lactamica | 3272 | + |
| Neisseria mucosa | 112 | − |
| Neisseria mucosa | 114 | + |
| Neisseria mucosa | 2888 | − |
| Neisseria subflava | 124 | + |
| Neisseria perflava | 120 | − |
| Neisseria sicca | 2844 | − |
| Neisseria sicca | 118 | + |
| Neisseria elongata | 129 | − |
| Neisseria cinerea | 126 | − |
| Neisseria cinerea | 2199 | − |
| Neisseria flava | 122 | − |
| Neisseria flava | 123 | + |
| Neisseria denitrificans | 2950 | − |
| Haemophilus influenzae | | − |
| Haemophilus parainfluenzae | | − |
| Streptococcus salivarius | | − |
| Streptococcus mutans | | − |
| Streptococcus agalactiae | | − |
| Staphylococus aureus | | − |
| Staphylococus epidermidis | | − |
| Staphylococus saprophyticus | | − |
| Escherichia coli | | − |

7

Tabelle IIIc: Proben, die spezifisch sind für N.gonorrhoeae

| Species/Stämme | | Nukleinsäure-Sonde |
|---|---|---|
| | | IgA 1* |
| Neisseria | 74 | + |
| gonorrhoeae | 514 | + |
| | 510 | + |
| | R2 | + |
| | R21 | + |
| | R 16 | + |
| | MS11 | + |
| Neisseria | B | – |
| meningitidis | C | – |
| | D | – |
| | Y | – |
| | W | – |
| | Z | – |
| | 8213 | – |
| Neisseria lactamica | 1855 | – |
| Neisseria lactamica | 2879 | – |
| Neisseria lactamica | 3272 | – |
| Neisseria mucosa | 112 | – |
| Neisseria mucosa | 114 | – |
| Neisseria mucosa | 2888 | – |
| Neisseria subflava | 124 | – |
| Neisseria perflava | 120 | – |
| Neisseria sicca | 2844 | – |
| Neisseria sicca | 118 | – |
| Neisseria clongata | 129 | – |
| Neisseria cinerea | 126 | – |
| Neisseria cinerea | 2199 | – |
| Neisseria flava | 122 | – |
| Neisseria flava | 123 | – |
| Neisseria denitrificans | 2950 | – |
| Haemophilus influenzae | | – |
| Haemophilus parainfluenzae | | – |
| Streptococcus salivarius | | – |
| Streptococcus mutans | | – |
| Streptococcus agalactiae | | – |
| Staphylococcus aureus | | – |
| Staphylococcus epidermidis | | – |
| Staphylococcus saprophyticus | | – |
| Escherichia coli | | – |

Tabelle IIId: Proben, die spezifisch sind für N.meningitidis

| Species/Stämme | | Nukleinsäure-Sonde Opa 3* |
|---|---|---|
| Neisseria gonorrhoeae | 74 | − |
|  | 514 | − |
|  | 510 | − |
|  | R2 | − |
|  | R21 | − |
|  | R 16 | − |
|  | MS11 | − |
| Neisseria meningitidis | B | + |
|  | C | + |
|  | D | + |
|  | Y | + |
|  | W | + |
|  | Z | + |
|  | 8213 | + |
| Neisseria lactamica | 1855 | − |
| Neisseria lactamica | 2879 | − |
| Neisseria lactamica | 3272 | − |
| Neisseria mucosa | 112 | − |
| Neisseria mucosa | 114 | − |
| Neisseria mucosa | 2888 | − |
| Neisseria subflava | 124 | − |
| Neisseria perflava | 120 | − |
| Neisseria sicca | 2844 | − |
| Neisseria sicca | 118 | − |
| Neisseria clongata | 129 | − |
| Neisseria cinerea | 126 | − |
| Neisseria cinerea | 2199 | − |
| Neisseria flava | 122 | − |
| Neisseria flava | 123 | − |
| Neisseria denitrificans | 2950 | − |
| Haemophilus influenzae | | − |
| Haemophilus parainfluenzae | | − |
| Streptococcus salivarius | | − |
| Streptococcus mutans | | − |
| Streptococcus agalactiae | | − |
| Staphylococus aureus | | − |
| Staphylococus epidermidis | | − |
| Staphylococus saprophyticus | | − |
| Escherichia coli | | − |

**Patentansprüche**

**Patentansprüche für folgende Vertragsstaaten : AT, BE, CH, DE, DK, FR, GB, GR, IT, LI, LU, NL, SE**

1. Neisseria-spezifische DNA-Sonde,
   **dadurch gekennzeichnet,** daß sie aus der Gruppe der folgenden Oligonukleotide

| Bezeichnung | Sequenz 5'->3' |
|---|---|
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| Iga 1 | cggtattacccggt |
| Opa 3 | gcagcccgtattat |

oder hierzu komplementären Sequenzen ausgewählt ist.

2. Sonde nach Anspruch 1, **dadurch gekennzeichnet,** daß sie am 5'- und/oder 3'-Ende bis zu 5 weitere Nukleotide aufweist.

3. Sonde nach Anspruch 2, **dadurch gekennzeichnet,** daß die weiteren Nukleotide den Nukleotiden entsprechen, die an dieser Stelle im natürlichen Gen, von dem die Sonden abgeleitet sind, vorhanden sind.

4. Sonde nach Anspruch 2 oder 3,
**dadurch gekennzeichnet,** daß sie aus der Gruppe der folgenden Oligonukleotide

| Bezeichnung | Sequenz 5'->3' |
|---|---|
| PIII* | ccatttccctgtctgccaa |
| Pil 1* | aaccggctgtccgcagaa |
| Iga 2* | cggacggtgcacaaattg |
| Opa 1* | ctgcgctgcggaagagaagag |
| Opa 2* | gcggcccgtatgtgcagg |
| Iga 1* | cggtattacccggttgt |
| Opa 3* | gcagcccgtattatgtgc |

oder hierzu komplementären Sequenzen ausgewählt ist.

5. Verfahren zum Nachweis wenigstens einer der pathogenen Neisseria-Spezies N. gonorrhoeae und N. meningitidis unter Anwendung einer hybridisierenden Sonde unter Hybridisierungsbedingungen und Nachweis der Hybridbildung, **dadurch gekennzeichnet,** daß man zum spezifischen Nachweis eine Sonde nach einem der Ansprüche 1 bis 4 verwendet.

6. Verfahren nach Anspruch 5, **dadurch gekennzeichnet,** daß man zum gemeinsamen Nachweis von N. gonorrhoeae und N. meningitidis mindestens eine der Sonden PIII, Pil 1, IgA 2 und Opa 1 verwendet.

7. Verfahren nach Anspruch 6, **dadurch gekennzeichnet,** daß man mindestens eine der Sonden PIII*, Pil1*, IgA2* und Opa1* verwendet.

8. Verfahren nach Anspruch 5, **dadurch gekennzeichnet,** daß man zum alleinigen Nachweis von N. gonorrhoeae mindestens eine der Sonden Opa 2, IgA 1 und insbesondere Opa2* und IgA1* verwendet.

9. Verfahren nach Anspruch 5, **dadurch gekennzeichnet,** daß man zum alleinigen Nachweis von N. meningitidis die Sonde Opa 3 und insbesondere Opa3* verwendet.

10. Verfahren nach einem der Ansprüche 4 bis 9, **dadurch gekennzeichnet,** daß man die Hybridbildung über die Markierung der Sonde nachweist.

EP 0 404 161 B1

**Patentansprüche für folgenden Vertragsstaat : ES**

1.  Verfahren zum Nachweis wenigstens einer der pathogenen Neisseria-Spezies N. gonorrhoeae und N. meningitidis unter Anwendung einer hybridisierenden Sonde unter Hybridisierungsbedingungen und Nachweis der Hybridbildung,
    **dadurch gekennzeichnet,**
    daß man zum spezifischen Nachweis eine Sonde, die aus der Gruppe der folgenden Oligonukleotide

| Bezeichnung | Sequenz 5' -> 3' |
|---|---|
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| Iga 1 | cggtattacccggt |
| Opa 3 | gcagcccgtattat |

    oder hierzu komplementären Sequenzen ausgewählt ist, verwendet.

2.  Verfahren nach Anspruch 1,
    **dadurch gekennzeichnet,**
    daß die Sonde am 5'- und/oder 3'-Ende bis zu 5 weitere Nukleotide aufweist.

3.  Verfahren nach Anspruch 2,
    **dadurch gekennzeichnet,**
    daß die weiteren Nukleotide den Nukleotiden entsprechen, die an dieser Stelle im natürlichen Gen, von dem die Sonden abgeleitet sind, vorhanden sind.

4.  Verfahren nach Anspruch 2 oder 3,
    **dadurch gekennzeichnet,**
    daß die Sonde aus der Gruppe der folgenden Oligonukleotide

| Bezeichnung | Sequenz 5' -> 3' |
|---|---|
| PIII* | ccatttccctgtctgccaa |
| Pil 1* | aaccggctgtccgcagaa |
| Iga 2* | cggacggtgcacaaattg |
| Opa 1* | ctgcgctgcggaagagaagag |
| Opa 2* | gcggcccgtatgtgcagg |
| Iga 1* | cggtattacccggttgt |
| Opa 3* | gcagcccgtattatgtgc |

    oder hierzu komplementären Sequenzen ausgewählt ist.

5.  Verfahren nach Anspruch 1,
    **dadurch gekennzeichnet,**
    daß man zum gemeinsamen Nachweis von N. gonorrhoeae und N. meningitidis mindestens eine der Sonden PIII, Pil 1, IgA 2 und Opa 1 verwendet.

6.  Verfahren nach Anspruch 5,
    **dadurch gekennzeichnet,**
    daß man mindestens eine der Sonden PIII*, Pil1*, IgA2* und Opa1* verwendet.

7.  Verfahren nach Anspruch 1,
    **dadurch gekennzeichnet,**
    daß man zum alleinigen Nachweis von N. gonorrhoeae mindestens eine der Sonden Opa 2, IgA 1 und

11

insbesondere Opa2* und IgA1* verwendet.

8. Verfahren nach Anspruch 1,
   **dadurch gekennzeichnet,**
   daß man zum alleinigen Nachweis von N. meningitidis die Sonde Opa 3 und insbesondere Opa3* verwendet.

9. Verfahren nach einem der Ansprüche 4 bis 8,
   **dadurch gekennzeichnet,**
   daß man die Hybridbildung über die Markierung der Sonde nachweist.

**Claims**
**Claims for the following Contracting States : AT, BE, CH, DE, DK, FR, GB, GR, IT, LI, LU, NL, SE**

1. Neisseria-specific DNA probe,
   **wherein**
   it is selected from the group of the following oligonucleotides

| Notation | Sequence 5' → 3' |
|----------|------------------|
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| Iga 1 | cggtattacccggt |
| Opa 3 | gcagcccgtattat |

   or from sequences that are complementary to them.

2. Probe as claimed in claim 1, **wherein** it has up to 5 further nucleotides at the 5' end and/or 3' end.

3. Probe as claimed in claim 2, **wherein** the further nucleotides correspond to the nucleotides which are present at that site in the natural gene from which the probes are derived.

4. Probe as claimed in claim 2 or 3, **wherein** it is selected from the group of the following oligonucleotides

| Notation | Sequence 5' → 3' |
|----------|------------------|
| PIII* | ccatttccctgtctgccaa |
| Pil 1* | aaccggctgtccgcagaa |
| Iga 2* | cggacggtgcacaaattg |
| Opa 1* | ctgcgctgcggaagagaagag |
| Opa 2* | gcggcccgtatgtgcagg |
| Iga 1* | cggtattacccggttgt |
| Opa 3* | gcagcccgtattatgtgc |

   or from sequences that are complementary to them.

5. Method for the detection of at least one of the pathogenic Neisseria species N. gonorrhoeae and N. meningitidis using a hybridizing probe under hybridization conditions and detection of the hybrid formation, **wherein** a probe as claimed in one of the claims 1 to 4 is used for the specific detection.

6. Method as claimed in claim 5, **wherein** at least one of the probes PIII, Pil 1, IgA 2 and Opa 1 is used for the combined detection of N. gonorrhoeae and N. meningitidis.

7. Method as claimed in claim 6, **wherein** at least one of the probes PIII*, Pil1*, IgA2* and Opa1* is used.

8. Method as claimed in claim 5, **wherein** at least one of the probes Opa 2, IgA 1 and in particular Opa2* and IgA1* is used for the sole detection of N. gonorrhoeae.

9. Method as claimed in claim 5, **wherein** the probe Opa 3 and in particular Opa3* is used for the sole detection of N. meningitidis.

10. Method as claimed in one of the claims 4 to 9, **wherein** the hybrid formation is detected via the labelling of the probe.

**Claims for the following Contracting State : ES**

1. Method for the detection of at least one of the pathogenic Neisseria Species N. gonorrhoeae and N. meningitidis using a hybridizing probe under hybridization conditions and detection of the hybrid formation,
**wherein**
a probe selected from the group of the following oligonucleotides

| Notation | Sequence 5' → 3' |
|----------|------------------|
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| Iga 1 | cggtattacccggt |
| Opa 3 | gcagcccgtattat |

or from sequences that are complementary to them, is used for the specific detection.

2. Method as claimed in claim 1,
**wherein**
the probe has up to 5 further nucleotides at the 5' and/or 3' end.

3. Method as claimed in claim 2,
**wherein**
the further nucleotides correspond to the nucleotides which are present at that site in the natural gene from which the probes are derived.

4. Method as claimed in claim 2 or 3,
**wherein**
the probe is selected from the group of the following oligonucleotides

| Notation | Sequence 5' → 3' |
|----------|------------------|
| PIII* | ccatttccctgtctgccaa |
| Pil 1* | aaccggctgtccgcagaa |
| Iga 2* | cggacggtgcacaaattg |
| Opa 1* | ctgcgctgcggaagagaagag |
| Opa 2* | gcggcccgtatgtgcagg |
| Iga 1* | cggtattacccggttgt |
| Opa 3* | gcagcccgtattatgtgc |

or from sequences that are complementary to them.

EP 0 404 161 B1

**5.** Method as claimed in claim 1,
**wherein**
at least one of the probes PIII, Pil 1, IgA 2 and Opa 1 is used for the combined detection of N. gonorrhoeae and N. meningitidis.

**6.** Method as claimed in claim 5,
**wherein**
at least one of the probes PIII*, Pil1*, IgA2* and Opa1* is used.

**7.** Method as claimed in claim 1,
**wherein**
at least one of the probes Opa 2, IgA 1 and in particular Opa2* and IgA1* is used for the sole detection of N. gonorrhoeae.

**8.** Method as claimed in claim 1,
**wherein**
the probe Opa 3 and in particular Opa3* is used for the sole detection of N. meningitidis.

**9.** Method as claimed in one of the claims 4 to 8,
**wherein**
the hybrid formation is detected via the labelling of the probe.

**Revendications**
**Revendications pour les Etats contractants suivants : AT, BE, CH, DE, DK, FR, GB, GR, IT, LI, LU, NL, SE**

**1.** Sonde d'ADN spécifique de Neisseria, caractérisée en ce qu'elle est choisie dans le groupe des oligonucléotides suivants:

| Dénomination | Séquence 5' -> 3' |
| --- | --- |
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| IgA 1 | cggtattacccggt |
| Opa 3 | gcagcccgtattat |

ou des séquences complémentaires de ceux-ci.

**2.** Sonde selon la revendication 1, caractérisée en ce qu'elle comporte jusqu'à 5 nucléotides supplémentaires à l'extrémité 5' et/ou 3'.

**3.** Sonde selon la revendication 2, caractérisée en ce que les nucléotides supplémentaires correspondent aux nucléotides qui sont présents à cet endroit dans le gène naturel dont dérivent les sondes.

**4.** Sonde selon la revendication 2 ou 3, caractérisée en ce qu'elle est choisie dans le groupe des oligonucléotides suivants:

| Dénomination | Séquence 5' -> 3' |
|---|---|
| PIII* | ccatttccctgtctgccaa |
| Pil 1* | aaccggctgtccgcagaa |
| IgA 2* | cggacggtgcacaaattg |
| Opa 1* | ctgcgctgcggaagagaagag |
| Opa 2* | gcggcccgtatgtgcagg |
| IgA 1* | cggtattacccggttgt |
| Opa 3* | gcagcccgtattatgtgc |

ou des séquences complémentaires de ceux-ci.

5. Procédé de mise en évidence d'au moins l'une des espèces pathogènes de Neisseria N. gonorrhoeae et N. meningitidis par utilisation, dans des conditions d'hybridation, d'une sonde qui s'hybride et par mise en évidence de la formation d'hybrides, caractérisé en ce que l'on utilise pour la mise en évidence spécifique une sonde selon l'une des revendications 1 à 4.

6. Procédé selon la revendication 5, caractérisé en ce que, pour la mise en évidence conjointe de N. gonorrhoeae et de N. meningitidis, on utilise au moins l'une des sondes PIII, Pil 1, IgA 2 et Opa 1.

7. Procédé selon la revendication 6, caractérisé en ce que l'on utilise au moins l'une des sondes PIII*, Pil 1*, IgA 2* et Opa 1*.

8. Procédé selon la revendication 5, caractérisé en ce que, pour la mise en évidence de N. gonorrhoeae seulement, on utilise au moins l'une des sondes Opa 2, IgA 1 et en particulier Opa 2* et IgA 1*.

9. Procédé selon la revendication 5, caractérisé en ce que, pour la mise en évidence de N. meningitidis seulement, on utilise la sonde Opa 3 et en particulier Opa 3*.

10. Procédé selon l'une des revendications 4 à 9, caractérisé en ce que l'on met en évidence la formation d'hybrides par le biais du marqueur de la sonde.

**Revendications pour l'Etat contractant suivant : ES**

1. Procédé de mise en évidence d'au moins l'une des espèces pathogènes de Neisseria N. gonorrhoeae et N. meningitidis par utilisation, dans des conditions d'hybridation, d'une sonde qui s'hybride et par mise en évidence de la formation d'hybrides, caractérisé en ce que l'on utilise pour la mise en évidence spécifique une sonde qui est choisie dans le groupe des oligonucléotides suivants:

| Dénomination | Séquence 5' -> 3' |
|---|---|
| PIII | ccatttccctgtct |
| Pil 1 | aaccggctgtccgc |
| IgA 2 | cggacggtgcacaa |
| Opa 1 | ctgcgctgcggaag |
| Opa 2 | gcggcccgtatgtg |
| IgA 1 | cggtattacccggt |
| Opa 3 | gcagcccgtattat |

ou des séquences complémentaires de ceux-ci.

2. Procédé selon la revendication 1, caractérisé en ce que la sonde comporte jusqu'à 5 nucléotides supplémentaires à l'extrémité 5' et/ou 3'.

3. Procédé selon la revendication 2, caractérisé en ce que les nucléotides supplémentaires correspondent aux nucléotides qui sont présents à cet endroit dans le gène naturel dont dérivent les sondes.

4. Procédé selon la revendication 2 ou 3, caractérisé en ce que la sonde est choisie dans le groupe des oligonucléotides suivants:

| Dénomination | Séquence 5' -> 3' |
|---|---|
| PIII* | ccatttccctgtctgccaa |
| Pil 1* | aaccggctgtccgcagaa |
| IgA 2* | cggacggtgcacaaattg |
| Opa 1* | ctgcgctgcggaagagaagag |
| Opa 2* | gcggcccgtatgtgcagg |
| IgA 1* | cggtattacccggttgt |
| Opa 3* | gcagcccgtattatgtgc |

ou des séquences complémentaires de ceux-ci.

5. Procédé selon la revendication 1, caractérisé en ce que, pour la mise en évidence conjointe de N. gonorrhoeae et de N. meningitidis, on utilise au moins l'une des sondes PIII, Pil 1, IgA 2 et Opa 1.

6. Procédé selon la revendication 5, caractérisé en ce que l'on utilise au moins l'une des sondes PIII*, Pil 1*, IgA 2* et Opa 1*.

7. Procédé selon la revendication 1, caractérisé en ce que, pour la mise en évidence de N. gonorrhoeae seulement, on utilise au moins l'une des sondes Opa 2, IgA 1 et en particulier Opa 2* et IgA 1*.

8. Procédé selon la revendication 1, caractérisé en ce que, pour la mise en évidence de N. meningitidis seulement, on utilise la sonde Opa 3 et en particulier Opa 3*.

9. Procédé selon l'une des revendications 4 à 8, caractérisé en ce que l'on met en évidence la formation d'hybrides par le biais du marqueur de la sonde.